# EUROPEAN PATENT APPLICATION

(11) **EP 3 531 103 A1**
(43) Date of publication of application: **28.08.2019**
(21) Application number: 18157907.9
(22) Date of filing: 21.02.2018
(51) Int. Cl.: G01N 1/14, G01N 33/24

(54) **AN EXTRACTOR, A SAMPLER SYSTEM INCORPORATING SAME AND A METHOD OF ITS USE**

(71) Applicant: Waikato Institute of Technology, 3200 Hamilton (NZ)
(72) Inventor: HATTINGH, Gert Erasmus, 3793 Ngaruawahia (NZ)
(74) Representative: Evens, Paul Jonathan

(57) **Abstract**

The invention is a system and method for collecting, analysing, or storing liquid from a substrate of interest in order to identify the presence/absence of chemicals of interest or the relative quantity of the liquid in the substrate while the system is in situ. One important aspect of the invention is the use in the system of an extractor (104) with a collection surface that includes a mesh layer (110). This mesh layer (110), extending across the collection area, provides some structural integrity to the overall extractor (104). This ensures that liquid can still pass through the collection area and onto the system for analysis, while allowing the extractor (104) to maintain its structural integrity.

## Description

### TECHNICAL OF THE INVENTION

The present invention relates to an extractor, a sampler system incorporating the extractor, and a method of using same. The invention has particular application to the *in situ* collection of a liquid sample, such as water, from a substrate such as soil or clay.

### DESCRIPTION OF RELATED ART

When investigating the properties of a substrate, it is often desirable to measure certain parameters, such as the presence or absence of certain chemicals. This may affect the management of proposed use of the substrate.

For example, when assessing the properties of soils used for pasture, it is useful to determine the relative abundance of available nutrients. For a farmer, this can affect how much fertiliser should be applied to a particular area of pasture.

Another example in which monitoring of the presence or absence of particular chemicals of concern may be useful is in ensuring compliance with appropriate environmental regulations. This can have a bearing on proposed changes in uses of the land and whether such land use changes would be permitted.

The presence or absence of chemicals can be determined by analysing water passing through the substrate. As the water percolates through the substrate, it may leach any chemicals that may be present in the substrate. Collecting any water passing through the substrate and analysing it can thus identify any chemicals that may present in the substrate (or absent from the substrate, as the case may be).

What may also be of interest to persons managing a substrate is the water content itself. This may be indicative of the potential for drought conditions and by understanding the extent of the residual moisture content, appropriate remedial action, in the form of the application of water for irrigation, may be taken. However, in such conditions it can be difficult to extract a water sample of sufficient quantity to allow analysis for any chemicals of interest. Conversely, a high water content may mean that no irrigation of the substrate is required. This also means that it may be easier to obtain a water sample.

Conventional methods of collecting water from soils is relatively invasive, requiring the soil to be extensively disturbed. Typically, a hole is excavated and a soil moisture sensor such as a lysimeter positioned therein. The soil is then replaced.

This is not only affects the natural layering of the soil, which can play a role in the migration of rain water from the top soil through to the water table but is also problematic when repeated sampling over a period of time, in order to detect any changes in the amount of chemicals that is present or absent in the soil, is desired.

Ideally, the substrate needs to be allowed to subside but this is not possible if it is repeatedly disturbed for the purpose of sampling. This may well be mitigated if a large area is of interest; the substrate is sampled just the once at a particular location and a different location is then used each time for successive samples.

However, this is problematic when a relatively small land area is of interest, such as when there is a spot source of pollution (from example, a leaking pipe or the like). This means that it is not practical to simply obtain a sample of the substrate from a nearby location as this may not identify the source of the pollution.

Collection of water sample can also be a relatively manual and time consuming process. A technician needs to travel to the site of interest, which may be quite remote, in order to locate and position the relevant equipment. This process needs to be repeated every time further samples are required. Furthermore, there is an ongoing risk that the soil may contain an inadequate amount of water for a sufficient sample to be extracted.

Unless the context clearly requires otherwise, throughout the description and the claims, the words "comprise", "comprising", and the like will be understood to imply the inclusion of a stated element, integer or step, or group of elements integers or steps, but not the exclusion of any other element, integer or step, or group of elements, integers or steps.

It is an object of the present invention to address the foregoing problems or at least to provide the public with a useful choice.

Further aspects and advantages of the present invention will become apparent from the ensuing description which is given by way of example only.

### DISCLOSURE OF THE INVENTION

According to one aspect of the present invention, there is provided an extractor for extracting and collecting a sample of liquid from a substrate, wherein the extractor includes:
a collection surface;
a chamber beneath the collection surface; and
a conduit linking the chamber to a vacuum pump,
wherein the collection surface includes a structural mesh layer.

According to another aspect of the present invention, there is provided a sampler system for investigating one or more properties of a substrate, wherein the sampler system includes:
an analysis unit for investigating one or more properties of a substrate, and
one or more extractors for extracting and collecting a sample of liquid from a substrate,
characterised in that
at least one of the one or more extractors includes a collection surface; a chamber beneath the collection surface; and a conduit linking the chamber to a vacuum pump, wherein the collection surface of the extractor includes a structural mesh layer.

According to another aspect of the present invention, there is provided a method of using the sampler system for investigating one or more properties of a substrate, the sampler system substantially as described above, wherein the method includes the steps of:
a) collecting a water sample for a substrate with the one or more extractors; and
b) transferring the water sample to the analysis unit.

According to another aspect of the present invention, there is provided a sampler system for investigating one or more properties of a substrate, wherein the sampler system includes:
one or more extractors for extracting and collecting a sample of liquid from a substrate, and
a storage unit for storing the sample of liquid, and
characterised in that
at least one of the one or more extractors includes a collection surface; a chamber beneath the collection surface; and a conduit linking the chamber to a vacuum pump, wherein the collection surface of the extractor includes a structural mesh layer.

According to another aspect of the present invention, there is provided a method of using the sampler system for investigating one or more properties of a substrate, the sampler system substantially as described above, wherein the method includes the steps of:
a) collecting a water sample for a substrate with the one or more extractors; and
b) transferring the water sample to the storage unit.

The invention is a system and method for collecting, analysing, or storing liquid from a substrate of interest in order to identify the presence/absence of chemicals of interest or the relative quantity of the liquid in the substrate while the system is in situ. One important aspect of the invention is the use in the system of an extractor with a collection surface that includes a mesh layer. This mesh layer, extending across the collection area, provides some structural integrity to the overall extractor. This ensures that liquid can still pass through the collection area and onto the system for analysis, while allowing the extractor to maintain its structural integrity.

Reference shall now be made throughout the remainder of the specification to the substrate in which the invention is to be used as being soil. However, it should be understood that this is not meant to be limiting and the use of the invention with substrates such as clay or sand, or indeed a mixture of substrates, is also envisaged by the applicant.

In exemplary embodiments of the invention, the liquid is the water that is present in the soil being investigated.

As the water (typically rain water but possibly also irrigation water) percolates through the soil from its surface, it may leach and carry with it a dissolved (or undissolved as the case may be) chemical or chemicals of interest that may be present in or on the soil. These chemicals may be of interest to persons associated with the management of the soil as they can reflect certain properties of the soil. The water content of the soil itself may also be a reflection of certain properties of the soil.

Thus, identification of the presence or absence of chemicals in the soil of interest, and/or the water content of that soil, may be indicative of an issue which may need to be appropriately remedied.

It will be appreciated that the chemical or chemicals of interest will vary according to the requirements of the user.

For example, it may be that the invention is used to sample the soils of farm land in order to assess the presence and concentration of certain nutrients in the soil. This in turn may determine the quantity and composition of fertiliser to be applied to the soil in order to maximise pasture growth.

For example, if soil is found to be lacking in nitrogen, then a fertiliser with a high nitrogen content may be applied to compensate.

Alternatively, if the soil of the farm is found to have a low water content beneath the root zone (the depth to which the roots of vegetation penetrates the soil), then it may require additional irrigation.

The invention may also be used to help monitor compliance with regulatory requirements across a wide range of sectors including, but not limited to, food manufacturing, disposal of waste and so forth.

For example, the invention may be used to detect pollution from primary food processing plants such as abattoirs. Hormones are commonly used in animal husbandry to optimise growth and increase body mass of animal species of commercial value for their flesh. It is undesirable for these hormones to enter waterways and thus there may be a need to monitor the surrounds of an abattoir. Another example may be the detection of heavy metals which may be indicative of pollution from manufacturing facilities. A person skilled in the art will readily appreciate other applications in industries for which detection of the presence and / or relevant concentrations of chemicals with the present invention may be used.

It should be noted that while reference is largely made in the present specification to the chemicals of the soil as being of interest, there is also scope for the invention to be used to collect a water sample from the soil where it is the residual microorganisms within the sample that is of interest. For example, the presence of *e. coli* bacteria may be indicative of pollution in the form of human- or animal-derived sewage.

The invention, which shall now be referred to as a sampler system, includes two major elements, the first being an extractor and the second being an analysis unit. However, in some embodiments of the sampler system, the second element may be a storage unit for collected samples.

It is also conceivable that in some other embodiments of the invention, the sampler system will include an extractor, an analysis unit and a storage unit. These elements will now be discussed in turn.

### Extractor

The extractor should be understood to include a collection surface, chamber and a conduit draining the chamber.

The overall dimensions of the extractor are likely to vary depending on the requirements of the user and the environment in which the sampler system is to be used. However, it will be appreciated that the collection surface is likely to ultimately determine the footprint of the extractor and this will in turn depend on the area of the substrate to be sampled.

In exemplary embodiments of the invention, the collection surface and chamber may be located within an open-topped housing or the like.

The collection surface is a structure which defines the general extent of the area from which the water in the overlying soil is to be extracted. The structure may be of a number of configurations including, but not limited to, a sphere, an elongate, horizontal cylinder, or an inverted pyramid or conical structure. Thus it will be appreciated that the collection surface may have a depth rather than simply being two-dimensional.

Each configuration of collection surface has application to specific scenarios; for example, when a large area of substrate is to be assessed, an elongate horizontal cylinder, several metres in length, may be appropriate. Such a configuration would be suitable for use when a homogenous sample is acceptable to the user.

For more focussed sampling, an inverted pyramid, i.e. the pyramid is arranged with its tip lowermost, with a base of a limited cross-sectional area may be more suitable. Persons skilled in the art will readily appreciate suitable configurations for specific scenarios.

Of course, the nature of the soil and substrate may have a bearing on the size of the collection surface. Factors such as soil type or surface topography could impact on the selection of the collection area.

The chamber is beneath the collection surface is such that any moisture extracted from the substrate above the catchment area makes its way into the chamber by virtue of gravity and, as will be explained later, application of a vacuum within the chamber.

The chamber may take any suitable configuration but preferably narrows progressively to a base portion. Thus the chamber may take an inverted pyramid or conical configuration. This assists in the channelling any collected moisture.

The chamber may be formed from any suitable material, such as stainless steel or the like which is resistant to corrosion. It may also be formed using conventional moulding techniques from an appropriate plastics material, for example, Acrylonitrile Butadiene Styrene (ABS), or a material such as fibreglass or the like. Persons skilled in the art will readily appreciate other materials that may be suitable for use in forming the chamber, given the environment in which it is to be located. However, it is desirable that whatever material that is used, is chemically inert so as to not affect the properties of the collected sample.

In exemplary embodiments of the present invention, the collection surface and chamber are positioned within a housing, with at least part of the top surface of the housing being open to allow liquid to enter the interior of the housing and collection surface.

The housing should be understood to include at least a base and sides and will include an opening for the conduit that drains the chamber. The housing may include a top but it will be appreciated that as noted above, the top must be at least partially open to allow water to reach the collection surface of the extractor.

The housing may be formed from any suitable material, such as stainless steel or the like which is resistant to corrosion. It may also be formed from an appropriate plastics material such as ABS or another material such as fibreglass or the like. Persons skilled in the art will readily appreciate other materials that may be suitable for use in forming the housing, given the environment in which it is to be located and the need to avoid the material used from affecting the properties of the collected sample.

To drain the chamber, a conduit extends from its base portion to the analysis unit and/or storage unit. This will be achieved in a variety of ways including, but not limited to, tubing or piping linking the extractor and analysis unit and/or storage unit.

In exemplary embodiments of the present invention, the conduit is stainless steel tubing of 1 millimetre (mm) diameter. This has sufficient structural integrity to resist some deformation that may occur during installation and when placed under a vacuum. However, this choice of material is not meant to be limiting and other types, preferably inert, may be used. Likewise, the stipulated dimension is not meant to be limiting and the selected tubing may be smaller or larger as the case may be.

The collection surface includes a structural mesh layer. This should be understood to be a mesh that provides some structural integrity to the overall structure.

In exemplary embodiments of the present invention, the structural mesh layer is positioned between the top of the collection surface and the chamber is a structural mesh layer.

It will be appreciated that in use, the sampler system is buried in the substrate and has soil overlying it. The structural mesh layer needs to have sufficient structural integrity to resist deformation brought about by the weight of the overlying substrate (plus any structures or objects placed permanently or intermittently on the substrate, or even animals and people moving about on the surface of the substrate). It also needs to be sufficiently robust to resist the creation of a suction or vacuum effect within the chamber of the extractor.

In exemplary embodiments of the present invention, the structural mesh layer is formed from stainless steel, forming a grid of 0.1 mm.

However, this material, and size, is not meant to be limiting. The structural mesh layer may alternatively be formed from suitable robust plastics material, such as fibreglass or toughened plastics. As previously noted with respect to the other components of the extractor, it is preferable that, regardless of what material is used, that the structural mesh layer be chemically inert.

In exemplary embodiments of the present invention, the collection area may include one or more of an additional layer of very fine mesh to assist with the removal of any particulate matter that may be present in the water as it percolates through the collection area and the structural mesh layer.

In some embodiments, for additional filtration of water, a layer of sand or similar inert filter material may be included in the collection area. This can also assist in the removal of particulate matter.

### Analysis unit

The sampler system may include an analysis unit. In this embodiment, the analysis unit is linked or is otherwise communicative with the extractor. In some embodiments, the analysis unit may be linked and communicative with more than one extractor.

The analysis unit should be understood to include an analysis station to perform measurement and testing of the water sample collected by the extractor. This may include, but is not limited to, measuring the volume of the collected water, the time in which a specified volume is collected, and analysing the collected water for the presence or absence of chemicals of interest.

Therefore, the analysis station will be understood to include apparatus that is readily automated to perform measurements and/or sense certain properties of the collected sample. For example, the analysis station could include pH sensors (to measure acidity/alkalinity). Alternatively, the analysis station could include means to administer reagents to the collected sample with appropriate detection means to assess any changes in the collected sample following administration of the reagent.

Also, as previously noted, in some embodiments of the present invention, it is the microorganisms within the soil that may be of interest. In such embodiments, the analysis station will obviously be provided with appropriate means to detect the presence and / or quantity of such microorganisms.

It should be understood that the above mentioned apparatus are just examples and are not intended to be limiting; persons skilled in the art will readily appreciate suitable apparatus that will be included in the analysis station depending on the nature of the analysis that is to be performed.

In exemplary embodiments of the present invention, the analysis unit is contained within its own housing. This means that if necessary, for example for maintenance purposes, the analysis unit may be independently removed from the extractor and replaced. A consequence of this is that the extractor can be left *in situ* if need be without disrupting the substrate immediately overlying it. If this substrate is disrupted or disturbed, this may affect the quality of the subsequent data.

However, it is not beyond the scope of the present invention, that the extractor and analysis unit be located within the same housing.

It will be appreciated though that this will have the disadvantage that to carry out any maintenance, the sampler system as a whole will need to be removed from the substrate, thus potentially disturbing the overlying soil. However, this may be mitigated by excavating the soil immediately adjacent to the sampler system and extracting it laterally.

In exemplary embodiments of the invention, the housing of the analysis unit is configured to allow the conduit from the extractor to pass and enter the analysis unit. This may require the use of appropriate seals and such like.

In exemplary embodiments of the present invention, within the analysis unit is provided a vacuum unit that includes a means to create a partial vacuum within the conduit and thus the chamber and collection area of the extractor.

However, in some embodiments of the present invention, it is envisaged that the vacuum unit to create the partial vacuum may be positioned within the housing of the extractor. This may not be ideal, as in order to perform any maintenance on the vacuum unit, the entire extractor will need to be removed and this will disturb the substrate.

The vacuum unit includes a means to generate a partial vacuum such as a vacuum pump, peristaltic pump or other device that creates a vacuum or suction effect. Reference shall now be made throughout the remainder of the present specification to the means for creating a partial vacuum as being a vacuum pump.

It should be understood that a vacuum reduces the boiling point of water. This means that there is the potential for the water collected to evaporate under application of a vacuum. This will be undesirable given the purpose of the present invention and thus persons skilled in the art will appreciate that this may have a bearing on the selection of the vacuum pump.

The inventor has used a vacuum pump typically found in trucks although this is not meant to be limiting and a person skilled in the art will readily identify suitable vacuum pumps that will create a sufficient vacuum in order to extract water from the substrate overlying the extractor. A suitable vacuum pressure is 500 millibar (mbar) or 15 millimetres of mercury (mmHg). At this pressure, the boiling point of water is 80°C and thus there is little risk of the collected sample being evaporated.

Other characteristics that may have a bearing on the selection of an appropriate vacuum pump are size, low power consumption, and maintenance requirements.

The vacuum pump should be understood to include or be linked to a chamber into which the water is drawn from the chamber of the extractor via the conduit.

In exemplary embodiments, sensors are present within the vacuum chamber to indicate the relative level of the water collected from the substrate.

The rate of increase, during the time periods the vacuum pump is activated, may be indicative of the overall water content of the substrate. This can be important as there may be a limit as to how much water can be extracted from the substrate, and a slow rate of increase may be indicative of conditions that may not be conducive to obtaining a large sample.

In some embodiments, sensors, or additional sensors to those of the chamber of the vacuum pump, may be positioned in the conduit in order to measure the amount of water passing through at any one time.

The vacuum chamber may include means to drain the collected water, or at least some of the collected water, to the analysis station for further processing. In some embodiments of the present invention, when only the amount of water content in the substrate is being assessed, the collected water may be drained to the exterior of the sampler system once its volume has been assessed.

The conduits between the vacuum pump and the chamber of the vacuum pump may be controlled through the use of valves. These valves may be automatically operable by an onboard operating means in the form of a central processing unit (CPU) or programmable logic controller (PLC), as will be discussed further below.

Likewise, the conduit extending between the chamber of the extractor and the analysis unit may be governed by a valve, preferably where the conduit enters the analysis unit. This means that should this valve need to replaced or repaired, this can be achieved with minimal disturbance of the extractor.

As noted above, in exemplary embodiments of the present invention, the analysis unit may be provided with an operating means in the form of a CPU or PLC for automatic operation of the sampler system.

This operating means controls the steps of the process of extracting the water sample from the soil, through the appropriate operation of the vacuum pump, the opening/closing of relevant valves allowing exit of the collected sample from the extractor, its entry into the vacuum chamber, as well as the transfer of the collected sample to the analysis station (or storage unit as the case may be).

The operating means may be programmed to operate the analysis unit, and the vacuum pump therein, at specific and regular periods of time, as determined by the user (however, there is scope for this to be manipulated depending on the requirements of the user or external factors, as shall be discussed below).

Thus the CPU or PLC is communicative with the valves. They may also be communicative with the sensors of the vacuum chamber and, if present, in the conduit.

However, in some embodiments of the present invention, it may be the extractor that is provided with the CPU or PLC, or indeed, both the analysis unit and extractor may each have a CPU or PLC that are communicative with each other.

It is a possibility that the residual water content of the soil may be prohibitive to obtaining a sufficient sample of water. In some embodiments of the invention, the CPU or PLC of the extractor and / or analysis unit may be communicative with soil moisture sensors that may be present in the soil nearby. Feedback from the soil moisture sensors may determine whether the extractor becomes operative (or not) in order to collect a sample.

In exemplary embodiments of the invention, the sampler system includes a means of transmitting collected data to a remote central processing station in a laboratory or the like. This does away with the need for a person to retrieve the analysis unit at least to obtain the collected data. In some embodiments, the CPU or PLC of the sampler system may be configured to issue instructions to irrigation means depending on the analysis of the water content of the soil. In such embodiments the sampler system may include an antennae or the like.

In exemplary embodiments of the invention, the sampler system includes a means of receiving information from a laboratory, a weather station or even nearby soil moisture sensors. This may allow the functionality for the CPU or PLC to adjust the sampling periods and frequency in response to external factors, such as weather events. It may also allow the collected data to be correlated with such external factors. In such embodiments the sampler system may include an antennae or the like.

In exemplary embodiments of the invention, the sampler system includes a long life battery. In some embodiments, the battery may be connected or otherwise linked to a supplementary power source (such as solar panels) to allow it to be operable for an extended period of time.

However, this is not meant to be limiting and the sampler system may be connected to an external power source such as mains power from a nearby building (although it will be appreciated that if this is the case, its wiring would need to be integrated during installation of the sampler system).

Thus, the sampler system may be positioned *in situ* and left to allow the soil that was disturbed during installation to settle before measurements begin to be taken over an extended period of time.

### Storage unit

In some embodiments of the present invention, the sampler system may include a storage unit for the collected samples. This could be instead of, or in addition to, the analysis unit.

The storage unit should be understood to be a structure with an automated packaging or bottling station. This processes the collected samples and packages or bottles them individually for later retrieval and analysis.

It will be straightforward for persons skilled in the art to implement such a storage unit. It will be appreciated that this means that there may be a conduit between the vacuum chamber and the packaging station.

The CPU or PLC, whether on board the storage unit or extractor, will be programmed with the appropriate steps to collect the desired quantity of sample and package it appropriately.

In these embodiments, to minimise potential for disturbance of the soil while retrieving the storage unit, it is preferable to locate it a short distance from the extractor although it should be appreciated that this distance may be partially determined by the strength of the vacuum pump of the vacuum unit.

### Installation

To install the sampler system, it is required to excavate a hole of sufficient depth to locate the extractor and analysis unit (and / or storage unit as the case may be). To minimise soil disturbance, it may be appropriate to excavate a vertical hole for the analysis unit, and then extend a cavity in the sides of the hole for the extractor.

This may be achieved through the use of a core drill, such as is commonly used in geology. A soil core could be extracted from the substrate and put to one side while the analysis unit and extractor are positioned. The core could then be replaced, perhaps with a portion removed to allow for the height of the analysis unit and/or extractor.

Alternatively, holes could be excavated for both the extractor and the analysis unit separately and the soil replaced on top and left to settle. It will be appreciated that a channel would need to be excavated for the conduit linking the extractor and analysis unit.

When used with additional soil moisture sensors, these may need to be located and connected with the sampler system as appropriate.

For best analysis, it is preferable to ensure that the holes are excavated to a depth that is below the root zone of any overlying plants present in the substrate. However, this is not meant to be limiting and the sampler system may be located anywhere within the substrate, from just beneath the soil surface to the underlying water table.

In summary, the invention provides a number of advantages over existing arrangements for collecting moisture from soil, including;
- easy to install and operate remotely;
- can be left *in situ* to sample the substrate over an extended period of time and thus identify any trends of interest;
- when linked to external sources of information, such as weather stations, can correlate local or remote events of interest with changes in soils;
- functionality to sample water content of different types of substrates;
- functionality to analyse a variety of chemicals of interest;
- functionality to cater for sampling of water content from substrates in a range of scenarios, encompassing small to large catchment areas; or
- at the very least, offers the public a useful choice.

The advantages and features of the present invention will be appreciated and understood by those skilled in the art and further aspects of the present invention will become apparent from the following description which is given by way of example only and with reference to the accompanying drawings.

### BRIEF DESCRIPTION OF DRAWINGS

Figure 1 is a schematic of a first exemplary embodiment of the present invention;
Figure 2 is a schematic of a second exemplary embodiment of the present invention;
Figure 3 is a schematic of a third exemplary embodiment of the present invention; and
Figure 4 is a schematic of the vacuum of the present invention.

### DETAILED DESCRIPTION OF THE INVENTION

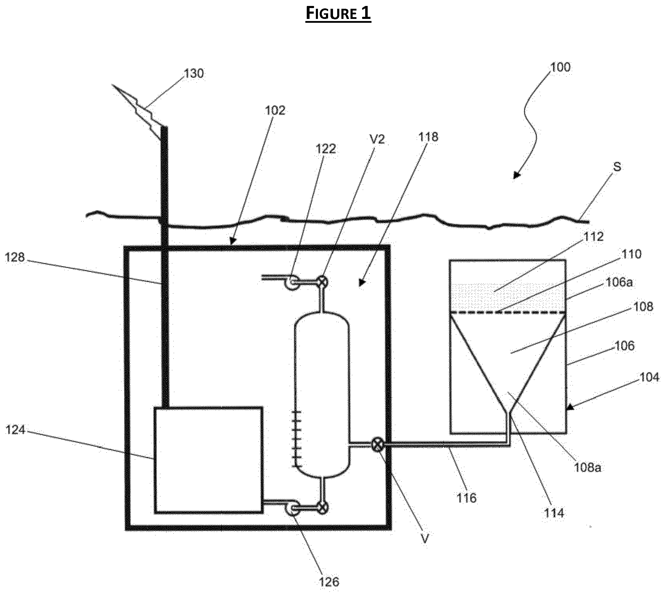

Figure 1 is a schematic of a first exemplary embodiment of the invention (generally indicated by arrow 100) in the form of a sampler system. The sampler system includes an analysis unit (102) and an extractor (104) both of which are located beneath the surface of the soil substrate (S).

Dealing first with the extractor, this includes an open-topped housing (106) that contains a chamber (108) over which is a collection surface including a structural mesh layer (110), which is rigid and relatively non-formable. This is necessary as the collection surface defines the upper side of the chamber.

Immediately overlying the structural mesh layer (110) is a inert filtering medium in the form of sand (112), which shall now be referred to as a sand filter, constrained by the upper part (106a) of the housing (106). Sand is non-adsorbent and does not retain water; it will simply pass around the particles of sand. This sand filter helps minimise any particulate matter being drawn into the chamber (108) as well as breaking up the potential for capillary action.

To accommodate the sand filter (112), the upper sides (106a) of the housing (106) are raised upwards of the collection surface.

It will be seen that the chamber (108) narrows progressively from its widest point, proximate the structural mesh layer (110), to its bottom (108a). This helps with the efficient conduction of the water as it is extracted from the soil substrate (S) towards the analysis unit (102).

At the bottom (108a) of the chamber (108) is provided an outlet (114) for a conduit (116). This conduit extends between the extractor (104) and the analysis unit (102). Inboard of the analysis unit, a valve (V1) is provided to shut off flow through the conduit if necessary. Ideally, the length of the conduit is kept to a minimum. This means that the distance that the collected water (not shown) has to travel from the chamber to the analysis unit is likewise kept to a minimum.

Within the analysis unit (102) is provided a vacuum unit (118), which includes a vacuum chamber (120) and a vacuum pump (122). Upon activation of the vacuum pump, this creates suction in the vacuum chamber, through the conduit (116) and into the chamber (108) of the extractor (104). This reduction of pressure within the vacuum chamber draws water down from the overlying soil substrate (S), through the sand filter (112) and structural mesh layer (110) of the extractor. The water (not shown) collects in the chamber of the extractor and is then drawn into the conduit and into the vacuum chamber of the vacuum unit.

When sufficient water (not shown) has been collected in the vacuum chamber (120) it can then be transferred to an analysis station (124) for assessment (or it could be discharged externally of the sampler system). The analysis station may be provided with its own pumping means in the form of a peristaltic pump (126), or a similar pumping mechanism, to achieve this. This transfer of the sample is done at normal pressure as to do so under vacuum may potentially damage the measuring and analysing equipment (not shown) within the analysis station.

A person skilled in the art will readily appreciate suitable measuring and analysing equipment (not shown) that would be used in the analysis station (124) and which would be appropriate for use with the present invention (100).

The analysis station (124) is also an ideal place to locate the central processing unit (CPU) (not shown) that automates the functioning of the sampler system (100). However, the CPU could just as easily be positioned elsewhere in the sampler system, including the extractor (104). Among other components, the CPU is communicative with the various valves (V1, V2, and V3) of the system, as well as the vacuum pump (122). It may also be communicative with external soil moisture sensors (not shown), which may determine whether or not to activate the vacuum pump in order to draw down a sample of water.

The sampler system (100) includes a wireless transmission means in the form of an antennae (128) to allow collected data (130) to be sent to a laboratory or the like (not shown). Although not shown in these figures, the sampler system is powered by a long-life battery or an external power source.

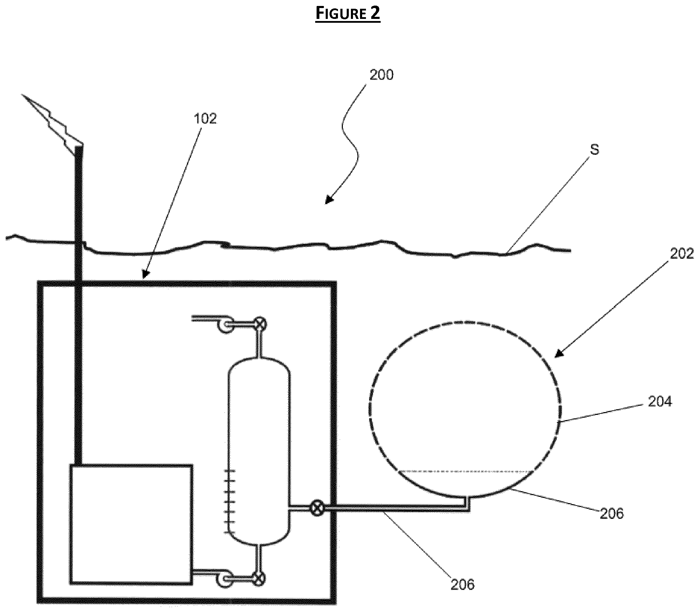

It should be appreciated that the extractor may take a variety of configurations depending on the nature and purpose of the analysis required. In the embodiment (200) of Figure 2, while the analysis unit (102) itself is as previously described, the extractor unit (202) is substantially spherical, with the majority of the surface area of the sphere being the structural mesh layer (204). Only at the base of the sphere (206) is the surface area continuous in order to direct the collected water towards the conduit (206).

This particular embodiment of the extractor (202) may be useful to maximise the collection of water from the soil substrate (S), drawing water not only from directly above the extractor but also from the substrate surrounding it. This could be particularly important in areas with relatively low rainfall and thus a drier substrate.

In contrast, the embodiment (100) of Figure 1 is more appropriate for focussed sampling of a smaller area of substrate, directly above the extractor (102).

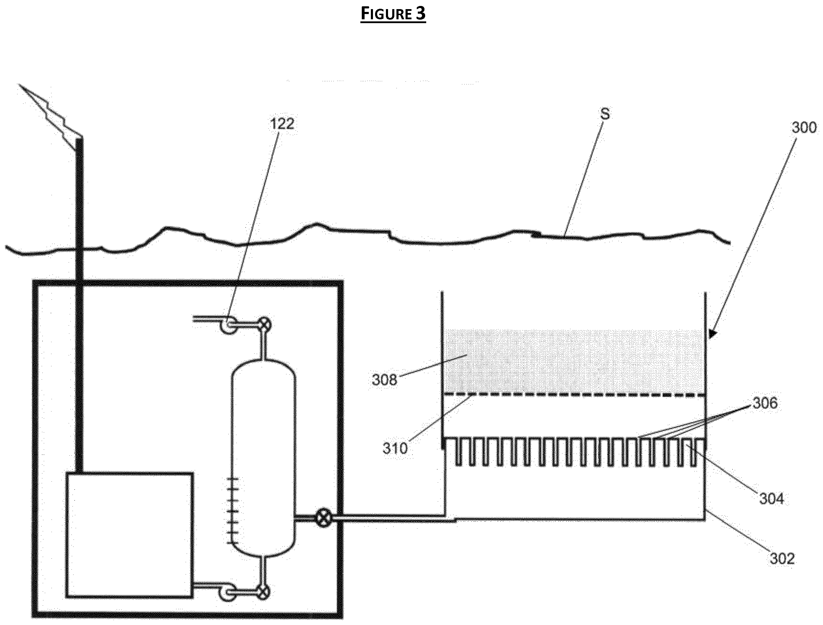

If sampling across a larger soil substrate is desired, then the extractor (300) may, as shown in Figure 3, take a substantially cylindrical form (302).

Here the upwards facing surface (304) of the cylinder (302) includes a plurality of slots (306) through which water passes as it is extracted from the overlying soil substrate (S). As with the embodiment of Figure 1, that of Figure 3 includes a sand filter (308) overlying a layer of structural mesh (310).

The cylinder (302) could be relatively long, up to 5 to 10 metres in length, although it will be appreciated that this may have a corresponding effect on the specifications of the vacuum pump (122) that will be required to generate the vacuum in order to extract the water from the soil substrate (S). Nonetheless, this configuration of extractor (300) may be useful for larger areas where homogeneity of the collected sample is important rather than the spot source sampling for which the embodiment (100) of Figure 1 is more suited.

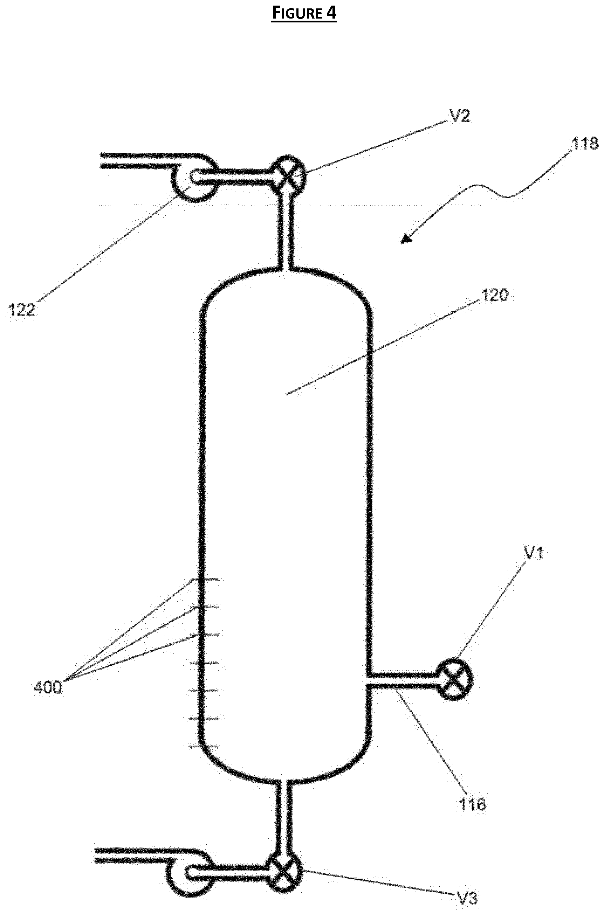

Figure 4 shows the vacuum pump (122) of the vacuum unit (118) of the analysis unit (not shown) of Figure 1. As can be seen, the vacuum chamber (120) includes a number of level sensors (400) to detect the amount of water therein. This can be useful when assessing the rate of water intake into the vacuum chamber (120) from the conduit (116) leading to the extractor (not shown). A rapid increase can be indicative of a soil that is relatively high in water content, particularly if it is over a prolonged period of time. Conversely, a slow rate of increase may be indicative of a soil with a low moisture content.

Valves control the conduits leading to the vacuum chamber (120); one (V2) is for the pump, another (V1) for the conduit leading to the chamber of the extractor, and the third (V3) for the outlet that leads to the analysis station (not shown). These are all operable by an onboard CPU (not shown).

Reference to any prior art in this specification is not, and should not be taken as, an acknowledgement or any form of suggestion that that prior art forms part of the common general knowledge in the field of endeavour in any country in the world.

The invention may also be said broadly to consist in the parts, elements and features referred to or indicated in the specification of the application, individually or collectively, in any or all combinations of two or more of said parts, elements or features.

Where in the foregoing description reference has been made to integers or components having known equivalents thereof, those integers are herein incorporated as if individually set forth.

It should be noted that various changes and modifications to the presently preferred embodiments described herein will be apparent to those skilled in the art. Such changes and modifications may be made without departing from the spirit and scope of the invention and without diminishing its attendant advantages. It is therefore intended that such changes and modifications be included within the present invention.

Aspects of the present invention have been described by way of example only and it should be appreciated that modifications and additions may be made thereto without departing from the scope thereof as defined in the appended claims.

## Claims

1. An extractor for extracting and collecting a sample of liquid from a substrate, wherein the extractor includes:
a collection surface;
a chamber beneath the collection surface; and
a conduit linking the chamber to a vacuum pump,
wherein the collection surface includes a structural mesh layer.

2. The extractor as claimed in claim 1, wherein the collection surface is a structure configured as one of the following:
• a sphere;
• a cylinder;
• an inverted pyramid; or
• an inverted cone.

3. The extractor as claimed in either claim 1 or claim 2, wherein the structural mesh layer is positioned between the collection surface and the chamber.

4. The extractor as claimed in any one of claims 1 to 3, wherein the structural mesh layer is formed from stainless steel.

5. The extractor as claimed in claim 4, wherein the structural mesh layer forms a grid of 0.1 mm.

6. The extractor as claimed in any one of claims 1 to 5, wherein the collection area includes an additional layer of fine mesh.

7. The extractor as claimed in any one of claims 1 to 6, wherein the collection surface and chamber is located within an open-topped housing having a base, sides, and an opening for a conduit that drains the chamber.

8. The extractor as claimed in claim 7, wherein the conduit is linked to a vacuum chamber and the vacuum pump.

9. A sampler system for investigating one or more properties of a substrate, wherein the sampler system includes:
one or more extractors as claimed in claim 1, and at least one of the following:
an analysis unit for investigating one or more properties of a substrate, wherein the analysis unit includes a vacuum pump; or
a storage unit for storing a sample of liquid extracted from the substrate being investigated.

10. The sampler system as claimed in claim 9, wherein the analysis unit includes an analysis station with apparatus for performing measurements and/or sensing certain properties of a collected sample.

11. The sampler system as claimed in either claim 9 or claim 10, wherein the analysis unit is contained in a housing separate from the one or more extractors, wherein the conduit of the one or more extractors enters the analysis unit.

12. The sampler system as claimed in claim 11, wherein the analysis unit includes a vacuum chamber linked via a conduit to the analysis station, wherein the analysis unit includes a pumping means to move a sample from the vacuum chamber to the analysis station.

13. The sampler system as claimed in any one of claims 9 to 12, wherein the system includes at least one of the following:
• an operating means in the form of a CPU or PLC for automatic operation of the sampler system;
• means of transmitting collected data to a remote central processing station;
• means of receiving information from one of the following: a laboratory, a weather station or soil moisture sensors linked to the system;
• a power source, wherein the power source is one of the following: a battery, mains power, or one or more solar panels; or
• a storage unit for collected samples.

14. The sampler system as claimed in claim 9, wherein the storage unit includes a vacuum pump.

15. A method of using a sampler system for investigating one or more properties of a substrate, wherein the sampler system is as claimed in claim 9, and wherein the method includes the steps of:
a) collecting a water sample for a substrate with the one or more extractors; and one of the following steps:
b) transferring the water sample to the analysis unit; or
c) transferring the water sample to the storage unit.
